# EUROPEAN PATENT SPECIFICATION

(11) **EP 3 584 950 B1**
(45) Date of publication and mention of the grant of the patent: **02.08.2023**
(21) Application number: 18906705.1
(22) Date of filing: 24.12.2018
(51) Int. Cl.: H04B 5/00, A61N 1/36, H02J 50/12, A61N 1/00

(54) **COMMUNICATION SYSTEM FOR IMPLANT DEVICE**
KOMMUNIKATIONSSYSTEM FÜR EINE IMPLANTATVORRICHTUNG
SYSTÈME DE COMMUNICATION POUR DISPOSITIF IMPLANT

(30) Priority: 20.04.2018 CN 201810357705
(43) Date of publication of application: 25.12.2019
(73) Proprietor: Hangzhou Nanochap Electronics Co., Ltd., Hangzhou, Zhejiang 311121 (CN)
(72) Inventor: YANG, Jiawei, Hangzhou, Zheijiang 311100 (CN)
(74) Representative: Wang, Bo
(86) International application number: PCT/CN2018/123078
(87) International publication number: WO 2019/200960

(56) References cited:
- EP-A2- 0 766 200
- CN-A- 102 157 989
- CN-A- 107 482 790
- CN-A- 107 769 812
- CN-A- 108 390 701
- CN-U- 208 386 541
- US-A- 5 630 835
- US-A1- 2008 082 147
- US-A1- 2012 244 802
- US-A1- 2017 361 113

## Description

### FIELD OF THE INVENTION

The present invention relates to the field of medical appliances, more particularly to a communication system of an implantable device and a communication method thereof.

### BACKGROUND OF THE INVENTION

An implant device generally uses coil coupling to transmit energy and signals, and traditional solutions generally use a structure of two coils, wherein one coil is disposed outside the body to transmit the signal, the other coil is disposed inside the body to receive the signal, and to archive charging of external to inside the body.

Since internal coupling coil L2 is restricted by size requirements, it is small in area and volume, while the size of the external coil L1 is not restricted by the installation, as a whole the coupling coefficient k12 is small, only by increasing the transmit power of L1, L2 can obtain more energy, so the voltage amplitude of the L2 coil is very large. After a forward signal is transmitted through L1 modulation, a signal with a certain envelop modulation depth (VPP_foward) is easily obtained on L2, this envelop depth fully satisfies demodulation requirements of the signal, thus forward communications can operate properly. Meanwhile, when sending reverse data transmission, there is a signal with amplitude modulation generates on the L2 coil, and a corresponding signal envelope (amplitude VPP_backward) appears on the L1 coil by load reflection, however, since k12 is very small, the amplitude of the useful signal envelope seen on L1 will be small. In some severe environments, the signal will be completely submerged by the signal and noise from L1, causing the receiver and demodulation unit in the external unit to fail to obtain the useful signal. Resulting in the reverse data work of the entire system failed.

Therefore, by simply using two coils is difficult to achieve effective transmission of forward and reverse data, and if the coupling coil of the reverse reception is further added in the external unit, it will be interfered by the coupling coil of the forward transmission signal, and effective transmission of the reverse signal is also difficult to achieve. US 2008/082147 A1 discloses a communication system of an implantable device, comprising an external unit and an implantable unit, and wherein the external unit and the implantable unit realize charging and bidirectional signal transmission of the external unit to the implantable unit by a wireless signal,wherein the external unit comprises a digital signal processing unit, a power transmission unit and a receiver and demodulation unit, the internal unit comprises a stimulation module and a signal receiver and transmission unit, the power transmission unit comprises a first coupling coil L1, the first coupling coil L1 is configured to transmit a signal to the internal unit, the signal receiver unit comprises a second coupling coil L2, the second coupling coil L2 is configured to receive the signal from the external unit and transmit a signal to the external unit, the receiver and modulation unit comprises a third coupling coil L3. Each of US 2012/244802 A1 and EP 0 766 200 A2 discloses that the coils on the two symmetrical parts are arranged to cross each other through a symmetric center, a cross point thereof overlaps with a center of the first coupling coil L1.

### BRIEF SUMMARY OF THE INVENTION

In order to solve the problem that reverse signal transmission work of traditional implanted devices is unfavorable, the present invention comprises two external coupling coils, wherein one is for transmitting the forward signal, and the other one is for receiving the reverse signal, and at the same time the shape and position setting of the two external coupling coils allows the reverse signal not to be interfered, thereby realizing the effective transmission of the bidirectional signal. The specific solutions are as follows:

A communication system of an implantable device is defined by claim 1. Preferred embodiments of the invention are stipulated in the dependent claims.

Beneficial effects of the present invention: two coupling coils are disposed outside the body, wherein one is for transmitting the forward signal, and the other one is for receiving the reverse signal, while the setting of the shape and position of the two coupling coils outside the body, are such that the reverse signal is not disturbed, thereby achieving effective transmission of the bidirectional signal. The problem that the signal has weak signal strength and tends to be interfered when the traditional implantable device transmits the signal in a reverse direction is overcome.

### BRIEF DESCRIPTION OF THE DRAWINGS

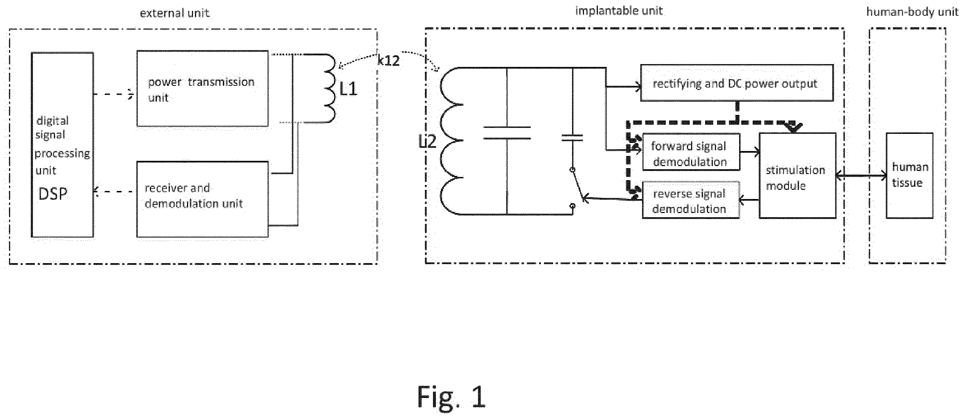

FIG. 1 is a communication schematic view of a traditional communication system;

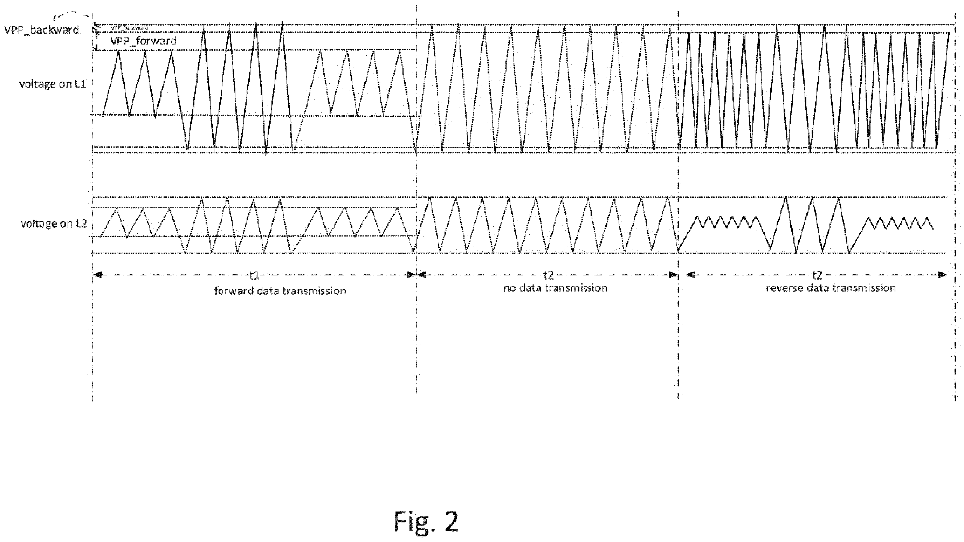

FIG. 2 is a communication waveform diagram of a traditional communication system;

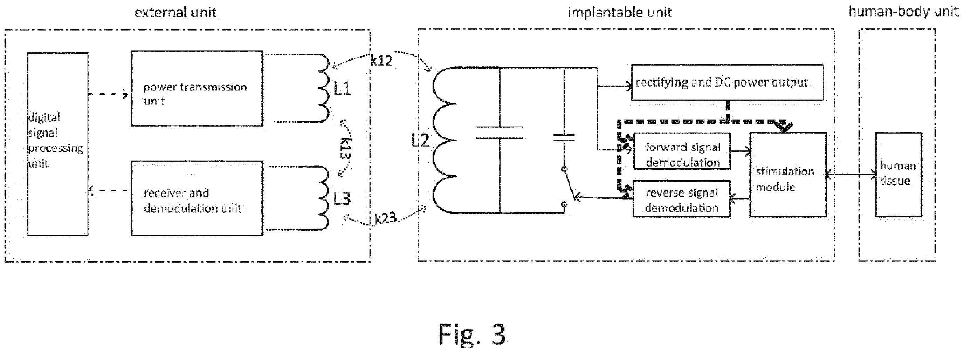

FIG. 3 is a communication schematic view of a communication system of the present intention;

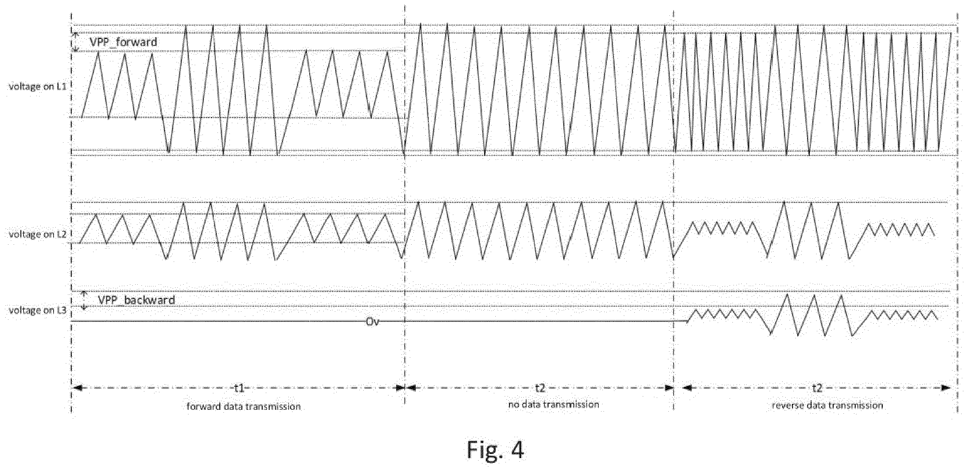

FIG. 4 is a communication waveform diagram of a communication system of the present invention;

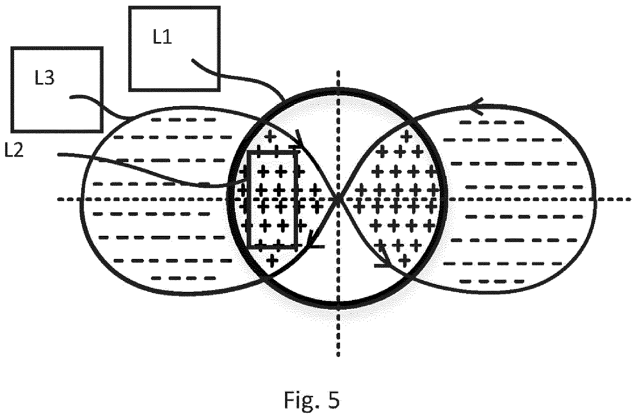

FIG. 5 is a schematic view showing the position and shape of a coil according to Example One of the present invention;

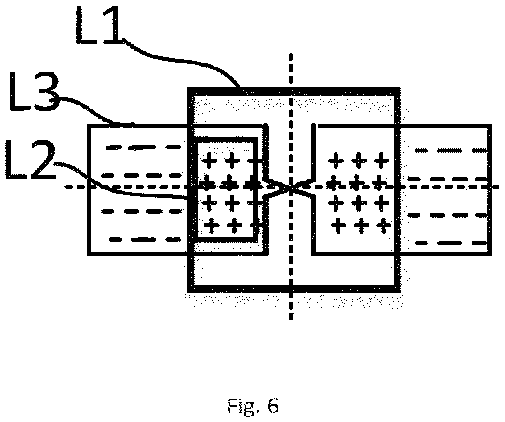

FIG. 6 is a schematic view showing the position and shape of a coil according to Example Two of the present invention;

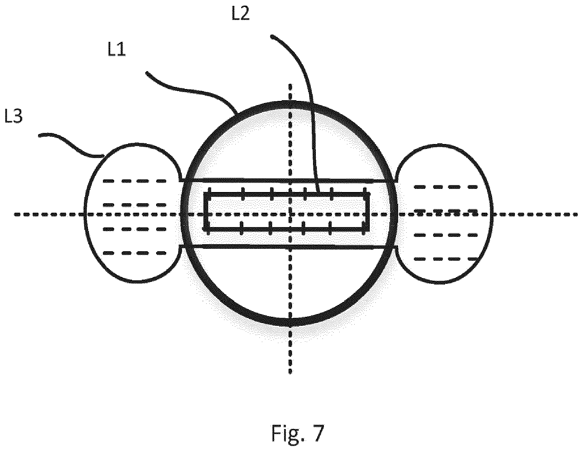

FIG. 7 is a schematic view showing the position and shape of a coil according to Example Three, not encompassed by the claimed invention.

### DETAILED DESCRIPTION OF EMBODIMENTS

In order to facilitate a further understanding of the present invention, it will be further illustrated below with reference to the accompanying drawings.

As shown in the drawings, the present invention firstly discloses a communication system of an implantable device, comprising: an external unit and an implantable unit, wherein the external unit and the implantable unit realize charging and bidirectional signal transmitting of the external unit to the implantable unit by a wireless signal. In which, the external unit comprises a digital signal processing unit, a power transmission unit and a receiver and demodulation unit, and the internal unit comprises a stimulation module and a signal receiver and transmission unit. The power transmission unit comprises a first coupling coil L1, the first coupling coil is configured to transmit a signal to the internal unit, the signal receiver unit comprises a second coupling coil L2, and the second coupling coil L2 is configured to receive the signal transmitted from the external unit and transmit a signal to the external unit, the receiver and modulation unit comprises a third coupling coil L3, and the third coupling coil is configured to receive the signal transmitted from the implantable unit. The third coupling coil L3 is disposed between the first coupling coil L1 and the second coupling coil L2.

During the signal transmission, the external unit transmits a signal to the internal unit via coupling between the first coupling coil and the second coupling coil in the internal unit, and the internal unit receives the signal and achieves charging.

Since the size of the second coupling coil L2 in the internal unit is generally small, in order for the internal unit to obtain more energy, it is necessary to increase the transmission power of the external unit, and it is necessary to increase the size of the first coupling coil in the external unit, and increase the coupling coefficient k12. If the first coupling coil is continuously used to receive the reverse signal transmitted from the internal unit, the reverse coupling coefficient k21 will be small, when the reverse data transmission is transmitted, there is a signal with an amplitude modulation on the second coupling coil, and a corresponding signal envelope (amplitude VPP_backward) appears on the L1 coil by load reflection, however, since coupling coefficient k21 is very small, the envelop amplitude of useful signal seen on the L1 will be very small, and in some severe environments, the signal will be completely submerged by the signal and the noise from L1, causing the receiver and demodulation unit in the external unit to fail to receive the useful signal, resulting in failure of the reverse data work of the entire system.

The present invention uses the third coupling coil L3 to receive the reverse signal, during the signal transmission, the center position of the first coupling coil L1 is aligned with the center position of the third coupling coil L3, and the third coupling coil L3 has a symmetrical shape, so that magnetic induction currents of the first coupling coil L1 coupled through the third coupling coil L3 are canceled each other out.

In general, the size of the first coupling coil L1 is larger than the size of the second coupling coil L2, and the projection of the second coupling coil L2 and the projection of the first coupling coil L1 overlap, ensuring the signal forwardly transmitted by the first coupling coil L1 will be effectively received by the second coupling coil L2 disposed in the body. Furthermore, the second coupling coil L2 is in the projection of the first coupling coil L1 and the third coupling coil L3, the coupling effect of the first coupling coil L1 and the second coupling coil L2 is better.

In addition, a signal shielding device for preventing interference with communications among the first coupling coil L1, the second coupling coil L2 and the third coupling coil L3 is further disposed outside the first coupling coil L1, and such signal shielding device can make the communications among the coupling coils more stable.

In order to prevent the reverse signal transmission from being affected by the first coupling coil and the second coupling coil, the position and shape setting of the third coupling coil can be implemented in various embodiments, and several are listed below.

### Example 1 (not covered by the claimed invention):

The third coupling coil L3 comprises two symmetrical parts, the coils on the two symmetrical parts are arranged to cross each other through a symmetric center, its cross point overlaps with the center of the first coupling coil, there is an offset between the center cross point position of the second coupling coil L2 and the third coupling coil L3, and positive and negative currents generated on the third coupling coil are canceled each other out, when the first coupling coil is coupled, and when the second coupling coil is coupled, the third coupling coil can fully receive the signal. In this Example, the first coupling coil is a toroidal coil, and the two symmetrical parts of the third coupling coil are both loop coils with a curvature.

### Example 2 (covered by the scope of the claimed invention):

The third coupling coil L3 comprises two symmetrical parts, the coils on the two symmetrical parts are arranged to cross each other through a symmetric center, its cross point overlaps with the center of the first coupling coil, there is an offset between the center cross point position of the second coupling coil L2 and the third coupling coil L3, and positive and negative currents generated on the third coupling coil are canceled each other out, when the first coupling coil is coupled, and when the second coupling coil is coupled, the third coupling coil can fully receive the signal. The first coupling coil is a square loop coil, and the two symmetrical parts of the third coupling coil are both square loop coils.

### Example 3 (not covered by the claimed invention):

The third coupling coil L3 comprises a region S 1 overlapping with the projection of the first coupling coil L1 and a region S2 not overlapping with the projection of the first coupling coil L1; through detecting and testing, a position of the third coupling coil relative to the first coupling coil is properly placed, so that the positive and negative magnetic fluxes passing through two regions of the third coupling coil L3 are canceled each other out, when the first coupling coil L1 is coupled. The third coupling coil L3 comprises an intermediate portion coil having a straight shape and end coils connected to the two ends of the intermediate portion, wherein the intermediate portion coil overlaps with the first coupling coil, and the two end coils does not overlap with the first coupling coil. When the signal is transmitted in a direction from the second coupling coil L2, the positive and negative magnetic fluxes passing through the overlap region S 1 and the non-overlap region S2 of the third coupling coil are equal, therefore when the signal is reversely transmitted, the coupling of the second coupling coil and the first coupling coil does not cause interference to the signal received by the third coupling coil.

The aforementioned examples are merely preferred examples of the present invention, which are not intended to limit the present invention.

## Claims

1. A communication system of an implantable device, comprising an external unit and an implantable unit, and wherein the external unit and the implantable unit realize charging and bidirectional signal transmission of the external unit to the implantable unit by a wireless signal,
wherein the external unit comprises a digital signal processing unit, a power transmission unit and a receiver and demodulation unit, the implantable unit comprises a stimulation module and a signal receiver and transmission unit, the power transmission unit of the external unit comprises a first coupling coil L1, the first coupling coil L1 is configured to transmit a signal to the implantable unit, the signal receiver unit of the implantable unit comprises a second coupling coil L2, the second coupling coil L2 is configured to receive the signal from the external unit and transmit a signal to the external unit, the receiver and modulation unit of the external device comprises a third coupling coil L3, and the third coupling coil L3 is configured to receive the signal from the implantable unit,
**characterized in that**,
the third coupling coil L3 comprises two symmetrical parts, the coils on the two symmetrical parts are arranged to cross each other through a symmetric center, a cross point thereof overlaps with a center of the first coupling coil L1, there is an offset between a center of the second coupling coil L2 and a center cross point position of the third coupling coil L3, and a positive and negative current generated by coupling the first coupling coil L1 through the third coupling coil L3 cancel each other out, and a size of the first coupling coil L1 is larger than a size of the second coupling coil L2, the second coupling coil L2 is in the projections of the first coupling coil L1 and the third coupling coil L3.

2. The communication system of the implantable device according to claim 1, **characterized in that**, the third coupling coil L3 is disposed between the first coupling coil L1 and the second coupling coil L2.

3. The communication system of the implantable device according to claim 1, **characterized in that**, a center position of the first coupling coil L1 is aligned with a center position of the third coupling coil L3, and the third coupling coil L3 has a symmetrical shape, the magnetic induction current of the first coupling coil L1 coupled through the third coupling coil L3 are canceled each other out.

4. The communication system of the implantable device according to claim 1, **characterized in that**, the first coupling coil L1 is a toroidal coil, and the two symmetrical parts of the third coupling coil L3 are both loop coils with a curvature.

5. The communication system of the implantable device according to claim 1, **characterized in that**, the first coupling coil L1 is a square loop coil, and the two symmetrical parts of the third coupling coil L3 are both square loop coils.

## Patentansprüche

1. Kommunikationssystem einer implantierbaren Einrichtung, welches eine externe Einheit und eine implantierbare Einheit umfasst, wobei die externe Einheit und die implantierbare Einheit ein Aufladen und eine bidirektionale Signalübertragung der externen Einheit zu der implantierbaren Einheit durch ein drahtloses Signal realisieren,
wobei die externe Einheit eine digitale Signalverarbeitungseinheit, eine Energieübertragungseinheit und eine Empfangs- und Demodulationseinheit umfasst, wobei die implantierbare Einheit ein Stimulationsmodul und eine Signal-Empfangs- und Übertragungseinheit umfasst, die Energieübertragungseinheit der externen Einheit eine erste Kopplungsspule L1 umfasst, die erste Kopplungsspule L1 dazu eingerichtet ist, ein Signal an die implantierbare Einheit zu übertragen, die Signal-Empfangseinheit der implantierbaren Einheit eine zweite Kopplungsspule L2 umfasst, die zweite Kopplungsspule L2 dazu eingerichtet ist, das Signal von der externen Einheit zu empfangen und ein Signal an die externe Einheit zu übertragen, die Empfangs- und Demodulationseinheit der externen Einrichtung eine dritte Kopplungsspule L3 umfasst und die dritte Kopplungsspule L3 dazu eingerichtet ist, das Signal von der implantierbaren Einheit zu empfangen,
**dadurch gekennzeichnet, dass**,
die dritte Kopplungsspule L3 zwei symmetrische Teile umfasst, die Spulen auf den zwei symmetrischen Teilen derart angeordnet sind, dass sie sich an einem Symmetriezentrum kreuzen, sich ein Kreuzungspunkt davon mit einem Mittelpunkt der ersten Kopplungsspule L1 überschneidet, ein Versatz zwischen einem Mittelpunkt der zweiten Kopplungsspule L2 und einer Mittelkreuzungspunkt-Position der dritten Kopplungsspule L3 vorliegt, sich ein positiver und ein negativer Strom, die durch Kopplung der ersten Kopplungsspule L1 durch die dritte Kopplungsspule L3 erzeugt werden, gegenseitig aufheben, die Größe der ersten Kopplungsspule L1 größer als die Größe der zweiten Kopplungsspule L2 ist und sich die zweite Kopplungsspule L2 in den Projektionen der ersten Kopplungsspule L1 und der dritten Kopplungsspule L3 befindet.

2. Kommunikationssystem der implantierbaren Einrichtung nach Anspruch 1, **dadurch gekennzeichnet, dass** die dritte Kopplungsspule L3 zwischen der ersten Kopplungsspule L1 und der zweiten Kopplungsspule L2 angeordnet ist.

3. Kommunikationssystem der implantierbaren Einrichtung nach Anspruch 1, **dadurch gekennzeichnet, dass** die Position der Mitte der ersten Kopplungsspule L1 mit der Position der Mitte der dritten Kopplungsspule L3 ausgerichtet ist, die dritte Kopplungsspule L3 eine symmetrische Form aufweist und sich die magnetisch induzierte Ströme der ersten Kopplungsspule L1, die durch die dritte Kopplungsspule L3 gekoppelt ist, gegenseitig aufgehoben sind.

4. Kommunikationssystem der implantierbaren Einrichtung nach Anspruch 1, **dadurch gekennzeichnet, dass** die erste Kopplungsspule L1 eine Toroidspule ist und die beiden symmetrischen Teile der dritten Kopplungsspule L3 jeweils Schleifenspulen mit einer Krümmung sind.

5. Kommunikationssystem der implantierbaren Einrichtung nach Anspruch 1, **dadurch gekennzeichnet, dass** die erste Kopplungsspule L1 eine quadratische Schleifenspule ist und die beiden symmetrischen Teile der dritten Kopplungsspule L3 jeweils quadratische Schleifenspulen sind.

## Revendications

1. Système de communication d'un dispositif implantable, comprenant une unité externe et une unité implantable, et dans lequel l'unité externe et l'unité implantable réalisent une charge et une transmission de signal bidirectionnel de l'unité externe vers l'unité implantable par un signal sans fil,
dans lequel l'unité externe comprend une unité de traitement de signal numérique, une unité de transmission de puissance et une unité de réception et de démodulation, l'unité implantable comprend un module de stimulation et une unité de réception et de transmission de signal, l'unité de transmission de puissance de l'unité externe comprend une première bobine de couplage L1, la première bobine de couplage L1 est configurée pour transmettre un signal à l'unité implantable, l'unité de réception de signal de l'unité implantable comprend une deuxième bobine de couplage L2, la deuxième bobine de couplage L2 est configurée pour recevoir le signal depuis l'unité externe et transmettre un signal à l'unité externe, l'unité de réception et de modulation du dispositif externe comprend une troisième bobine de couplage L3, et la troisième bobine de couplage L3 est configurée pour recevoir le signal depuis l'unité implantable,
**caractérisé en ce que**,
la troisième bobine de couplage L3 comprend deux pièces symétriques, les bobines sur les deux pièces symétriques sont agencées pour se croiser à travers un centre de symétrie, un point de croisement de celles-ci chevauche un centre de la première bobine de couplage L1, il existe un décalage entre un centre de la deuxième bobine de couplage L2 et une position de point de croisement central de la troisième bobine de couplage L3, et un courant positif et un courant négatif générés par couplage de la première bobine de couplage L1 à travers la troisième bobine de couplage L3 s'annulent, et une taille de la première bobine de couplage L1 est plus grande qu'une taille de la deuxième bobine de couplage L2, la deuxième bobine de couplage L2 est dans les saillies de la première bobine de couplage L1 et de la troisième bobine de couplage L3.

2. Le système de communication du dispositif implantable de la revendication 1, **caractérisé en ce que** la troisième bobine de couplage L3 est disposée entre la première bobine de couplage L1 et la deuxième bobine de couplage L2.

3. Le système de communication du dispositif implantable de la revendication 1, **caractérisé en ce que** une position centrale de la première bobine de couplage L1 est alignée avec une position centrale de la troisième bobine de couplage L3, et la troisième bobine de couplage L3 a une forme symétrique, les courants d'induction magnétique de la première bobine de couplage L1 couplée à travers la troisième bobine de couplage L3 s'annulent.

4. Le système de communication du dispositif implantable de la revendication 1, **caractérisé en ce que** la première bobine de couplage L1 est une bobine torique, et les deux pièces symétriques de la troisième bobine de couplage L3 sont toutes deux des bobines spirales avec une courbure.

5. Le système de communication du dispositif implantable de la revendication 1, **caractérisé en ce que** la première bobine de couplage L1 est une bobine spirale carrée, et les deux pièces symétriques de la troisième bobine de couplage L3 sont toutes deux des bobines spirales carrées.
